# EUROPÄISCHE PATENTANMELDUNG

(11) **EP 0 958 782 A1**
(43) Veröffentlichungstag der Anmeldung: **24.11.1999**
(21) Anmeldenummer: 99108727.1
(22) Anmeldetag: 03.05.1999
(51) Int. Cl.: A61B 5/107

(54) **Vorrichtung zum Vermessen von Gliedmassen**

(30) Priorität: 22.05.1998 DE 19822956
(71) Anmelder: Becker, Thomas P. Dr.-Ing., 66806 Ensdorf (DE)
(72) Erfinder: Becker, Thomas P. Dr.-Ing., 66806 Ensdorf (DE)
(74) Vertreter: Wieske, Thilo

(57) **Zusammenfassung**

Die Erfindung betrifft eine Vorrichtung zum Vermessen von Gliedmaßen, wobei bei der Vermessung wenigstens eine Aufnahme gemacht wird, anhand der Form und/oder Maße bestimmt werden; wobei die Vorrichtung wenigstens eine Kamera (1) enthält; die so positionierbar ist, daß neben den, zu vermessenden Gliedmaß wenigstens ein vorbekanntes Maß mit aufgenommen wird. Ebenso betrifft die vorliegende Erfindung ein Verfahren zum Vermessen von Gliedmaßen unter Verwendung einer der vorgenannten Vorrichtungen, wobei wenigstens die Kamera (1) in mehreren Positionen zum zu vermessenden Gliedmaß aufgestellt wird und wobei aus den in den einzelnen Positionen aufgenommenen Bildern die vollständige Form des Gliedmaßes bestimmt wird, indem die einzelnen Bilder in deren überlappenden Bereichen maßstabsgerecht so zusammengesetzt werden, daß sich die vollständige Form des Gliedmaßes ergibt.

## Beschreibung

Die Erfindung betrifft eine Vorrichtung zum Vermessen von Gliedmaßen nach dem Oberbegriff des Patentanspruches 1 sowie ein Verfahren nach dem Oberbegriff des Patentanspruches 3.

Es ist bereits eine Vorrichtung nach Anspruch 1 bekannt (DE 42 05 468 A1), bei der auf einen Körper ein bekanntes Maß aufprojiziert wird. Das bekannte Maß ändert sich dabei entsprechend der Oberfläche des Körpers. Aus der Änderung dieses Maßes, die mit einer Kamera ausgewertet wird, wird die Form des Körpers bestimmt.

Weiterhin ist eine Vorrichtung zur Vermessung eines Fußes bekannt, bei dem der Fuß aufgestellt wird. Weiterhin ist eine Lasermeßvorrichtung vorgesehen, die oberhalb des Fußes in Längsrichtung des Fußes verfahrbar ist. Die Lasermeßvorrichtung hält dabei in vordefinierten Punkten in vorbestimmten Abständen an, um einen Meßpunkt aufzunehmen. Die Lasermeßvorrichtung ist nicht nur in Längsrichtung des Fußes verfahrbar sondern auch um den Fuß drehbar. Durch eine Vielzahl von aufgenommen Meßpunkten kann damit die Oberfläche des Fußes bestimmt werden.

Es ist die Aufgabe der vorliegenden Erfindung, eine Vorrichtung und ein Verfahren vorzuschlagen, so daß eine vereinfachte Vermessung von Gliedmaßen möglich ist.

Diese Aufgabe wird erfindungsgemäß durch eine Vorrichtung nach Anspruch 1 gelöst, wonach die Vorrichtung wenigstens eine Kamera enthält, die so positionierbar ist, daß neben dem zu vermessenden Gliedmaß wenigstens ein vorbekanntes Maß mit aufgenommen wird.

Dadurch kann vorteilhaft auf eine feste Verbindung und eine feste Zuordnung der Kamera zu einem definierten Ort, an dem sich das zu vermessende Objekt befinden muß, verzichtet werden. Insgesamt wird die Vorrichtung dadurch wesentlich kleiner und kann beispielsweise auch einfach transportabel ausgestaltet werden. Dadurch ist es beispielsweise möglich, diese Vorrichtung in Krankenhäusern zu einem Patienten zu bringen. Der Patient muß sich dann nicht in einen besonderen Raum zur Untersuchung begeben.

Bei der Ausgestaltung der Vorrichtung nach Anspruch 2 besteht die Vorrichtung aus zwei Teilen, deren einer Teil eine Aufnahmeplatte aufweist, auf der das zu vermessende Gliedmaß positionierbar ist, wobei mit dieser Aufnahmeplatte wenigstens ein vorbekanntes Maß verbunden ist, und deren anderer Teil wenigstens eine um wenigstens eine Achse schwenkbare Kamera enthält.

Durch diese Aufteilung der Vorrichtung ist diese besonders einfach transportierbar. Weiterhin besteht eine große Flexibilität des Aufstellungsortes der Kamera.

Anspruch 3 betrifft ein Verfahren zum Vermessen von Gliedmaßen unter Verwendung einer der vorgenannten Vorrichtungen, wobei die Kamera in mehreren Positionen zum zu vermessenden Gliedmaß aufgestellt wird und wobei aus den in den einzelnen Positionen aufgenommenen Bildern die vollständige Form des Gliedmaßes bestimmt wird, indem die einzelnen Bilder in deren überlappenden Bereichen maßstabsgerecht so zusammengesetzt werden, daß sich die vollständige Form des Gliedmaßes ergibt.

Dieses Verfahren ist beispielsweise realisierbar, indem die von der Kamera aufgenommenen Bilder mittels einer geeigneten Software verarbeitet und zu einem geschlossenen Bild zusammengesetzt werden.

Das Verfahren und die Vorrichtung sind beispielsweise im Bereich der Orthopädie einsetzbar. Es ist beispielsweise möglich, in einem Schuhgeschäft eine Vermessung der Füße vorzunehmen, um eine Maßanfertigung vorzunehmen oder um einen passenden Schuh zu finden. Weiterhin ist es beispielsweise möglich, im Reha-Bereich Prothesen optimal anzupassen.

Ein Ausführungsbeispiel der Erfindung ist in der Zeichnung dargestellt. Es zeigt dabei im einzelnen:

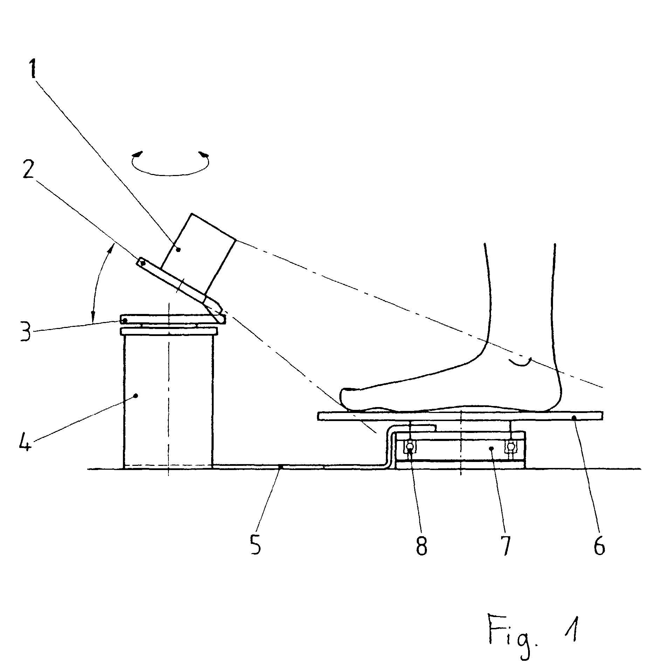

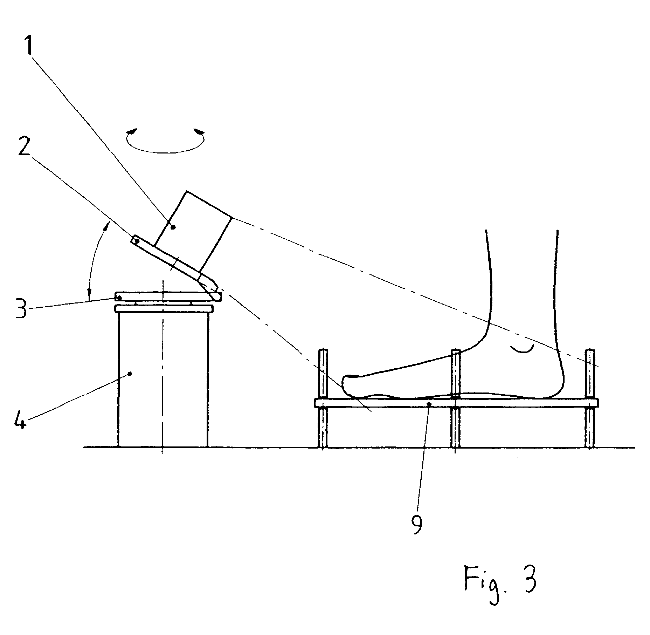

- Fig. 1 bis 6:: verschiedene Ausführungsbeispiele einer erfindungsgemäßen Vorrichtung.

In den Figuren sind gleiche Teile mit jeweils gleichen Bezugszeichen versehen.

Die Vorrichtung besteht aus fünf Teilen, dem Scanner 1, dem Träger mit der zugehörigen Winkelverstellung 2-3, der Basiseinheit mit einem motorischen Antrieb und ggf. einer Positionierhilfe sowie einer Standplatte für das zu vermessende Objekt.

Der Scanner 1 selbst besteht aus einer Linienlichtquelle und einer CCD-Kamera zur Detektion eines Schnittbildes, welches durch die Linienlichtquelle auf das zu messende Objekt projiziert wird. Der Scanner 1 mißt direkt die Entfernung zwischen Scanner, Basiseinheit und Objekt nach dem Prinzip der Triangulation, der Schnittbildmessung oder des Schattenwurfes.

Die Basiseinheit ist ein Drehgestell, das motorisch angetrieben ist. Der Träger 3 mit der Winkelverstellung 2 auf der Basiseinheit 4 ist fest mit dem Drehteil verbunden. Der darauf montierte Scanner 1 kann in einem großen Winkelbereich geneigt bzw. gekippt werden.

Die Positionierhilfe 5-7 besteht aus einem Ausleger 5, einem Drehteil 8, einem Standfuß 7 und einer Basisplatte 6 für die Aufnahme des Objektes.

Der Scanner 1 wirft sein Licht auf das zu vermessende Objekt und macht eine Momentaufnahme einer Kontur. Durch den motorischen Antrieb wird er um seine Achse kontinuierlich oder schrittweise gedreht und kann so vom Objekt mehrere Aufnahmen machen, aus denen eine dreidimensionale Fläche bestimmt werden kann, die ein Abbild des Objektes darstellt.

Um das Objekt vollständig zu vermessen, kann der Scanner 1 mit seiner Basiseinheit beliebig um das Objekt herum plaziert werden und das Objekt erneut vermessen werden. Ein fester Abstand zum Objekt kann durch die Positionierhilfe bewerkstelligt werden, in die die Basiseinheit gestellt werden kann. Durch die Winkelverkippung kann die optimale Scan-Position eingestellt werden oder durch weitere Verkippungen ein größeres Objekt vermessen werden als der Scan-Bereich des Scanners 1 es mit einer Einstellung zuläßt.

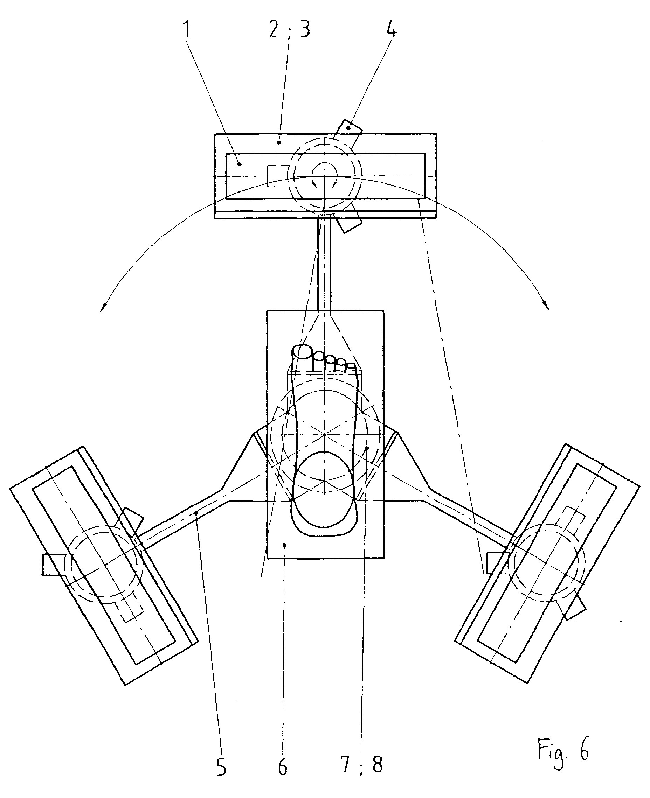

Die Positionierhilfe ist hier als Drehgestell ausgeführt mit einem standfesten Fuß und einer damit verbundenen Standfläche. Das Drehgestell hat einen drehbaren Arm mit ein bis zwei Stiften zur besseren Positionierbarkeit des Scanners mit seiner Basiseinheit. Gleichzeitig stellt das Drehgestell auch eine äußere Referenz dar, mit deren Hilfe das Objekt im Maßstab 1:1 vermessen werden kann (Figur 6).

Durch Kenntnis des Winkels um die Drehachse können die 3D-Koordinaten eines jeden Punktes auf dem Objekt bestimmt werden. Um das Objekt im Maßstab 1:1 zu vermessen, wird eine äußere Referenz benötigt, deren Ausmaße bekannt sind und deren Größenverhältnisse während der Messung mit vermessen werden können.

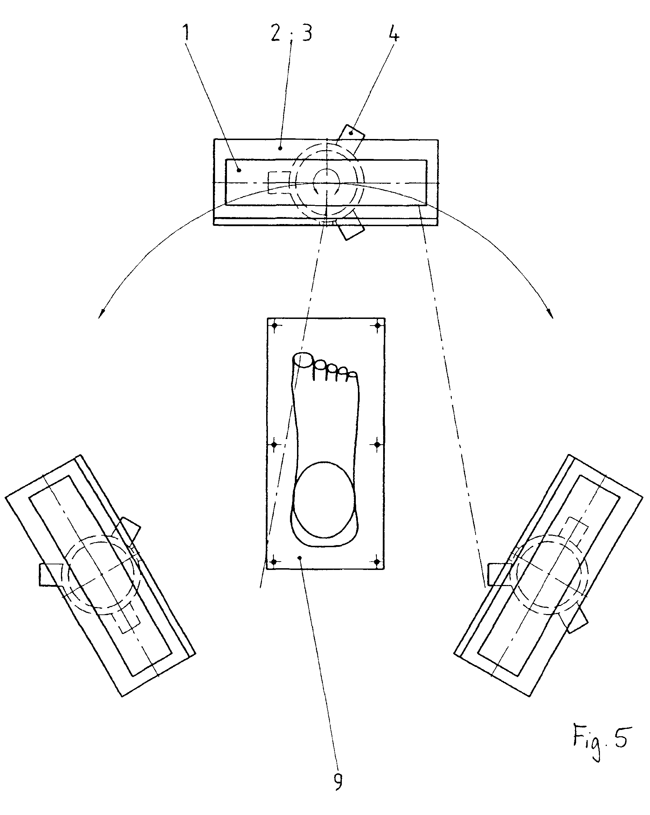

Eine weitere äußere Referenz stellt eine Basisplatte 9 mit einem oder mehreren Stiften bekannter Geometrie dar, wodurch der Scanner 1 frei ohne Verbindung zum Objekt verwendet werden kann und die Vorrichtung flexibler gestaltet wird (Figur 5).

Durch eine geeignete Meßsoftware kann aus mehreren Aufnahmen über die äußere Referenz das Gesamtobjekt zusammengesetzt werden.

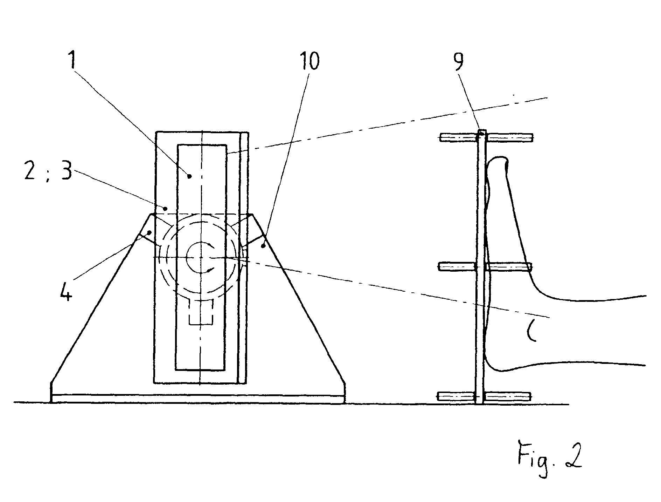

In Figur 2 ist die Vorrichtung um 90° um ihre Standachse gedreht und ruht auf einem zusätzlichen Ständer 10. Die Basisplatte 9, die in diesem Fall aus einem durchsichtigen Material ist, läßt beispielsweise das Scannen der Fußsohle oder der Handfläche zu. Auch ein Armstumpf oder ein Beinstumpf hinter der durchsichtigen Platte kann so vermessen werden.

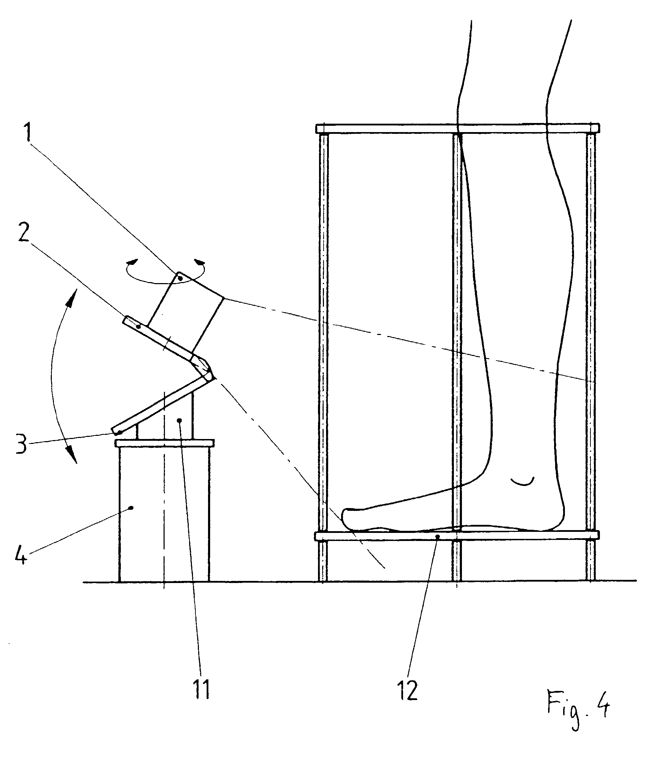

Mit einer größeren Referenz (Figur 4) aus Stäben und Platten 12 und gegebenenfalls einer erweiterten Kippeinrichtung 11 für den Scanner 1 lassen sich große Objekte oder längere Gliedmaßen vermessen. Die Stäbe dienen als Referenz, wenn ihre Maße bekannt sind.

Ein wesentlicher Vorteil dieser Vorrichtung ist ihre Kompaktheit, die dazu führt, daß die Vorrichtung tragbar und leicht transportierbar ist und an jedem beliebigen Ort leicht und ohne Anstrengung aufzubauen ist und durch eine leichte Handhabung des Gerätes auch zur Vermessung größerer Objekte benutzt werden kann.

## Patentansprüche

1. Vorrichtung zum Vermessen von Gliedmaßen, wobei bei der Vermessung wenigstens eine Aufnahme gemacht wird, anhand der Form und/oder Maße bestimmt werden,
dadurch gekennzeichnet, daß die Vorrichtung wenigstens eine Kamera (1) enthält, die so positionierbar ist, daß neben dem zu vermessenden Gliedmaß wenigstens ein vorbekanntes Maß (9, 12) mit aufgenommen wird.

2. Vorrichtung nach Anspruch 1,
dadurch gekennzeichnet, daß die Vorrichtung aus zwei Teilen besteht, deren einer Teil eine Aufnahmeplatte (9, 12) aufweist, auf der das zu vermessende Gliedmaß positionierbar ist, wobei mit dieser Aufnahmeplatte (9, 12) wenigstens ein vorbekanntes Maß verbunden ist, und deren anderer Teil wenigstens eine um wenigstens eine Achse schwenkbare Kamera (1) aufweist.

3. Verfahren zum Vermessen von Gliedmaßen unter Verwendung einer der vorgenannten Vorrichtungen,
dadurch gekennzeichnet, daß wenigstens die Kamera (1) in mehreren Positionen zum zu vermessenden Gliedmaß aufgestellt wird und daß aus den in den einzelnen Positionen aufgenommenen Bildern die vollständige Form des Gliedmaßes bestimmt wird, indem die einzelnen Bilder in deren überlappenden Bereichen maßstabsgerecht so zusammengesetzt werden, daß sich die vollständige Form des Gliedmaßes ergibt.
